# EUROPEAN PATENT SPECIFICATION

(11) **EP 3 007 747 B1**
(45) Date of publication and mention of the grant of the patent: **10.05.2017**
(21) Application number: 14729897.0
(22) Date of filing: 12.06.2014
(51) Int. Cl.: A61M 5/20, F16F 1/06, A61M 5/24, A61M 5/315

(54) **DRUG DELIVERY DEVICE WITH DUAL LAYER SPRING**
ARZNEIMITTELABGABEVORRICHTUNG MIT ZWEILAGIGER FEDER
DISPOSITIF D'ADMINISTRATION DE MÉDICAMENT AVEC RESSORT À DOUBLE COUCHE

(30) Priority: 14.06.2013 EP 13172031; 19.06.2013 US 201361836804 P
(43) Date of publication of application: 20.04.2016
(73) Proprietor: Novo Nordisk A/S, 2880 Bagsværd (DK)
(72) Inventor: KIILERICH, Ebbe, DK-2880 Bagsværd (DK)
(86) International application number: PCT/EP2014/062295
(87) International publication number: WO 2014/198858

(56) References cited:
- WO-A1-92/12358
- WO-A1-2006/045528
- WO-A1-2011/060786

## Description

### FIELD OF THE INVENTION

The present invention relates to power assisted drug delivery devices, such as e.g. automatic injection devices. The invention particularly relates to drug delivery devices employing a compact spring element for delivering energy to expel drug from a reservoir.

### BACKGROUND OF THE INVENTION

Drug delivery devices, such as injection devices, are widely used for administration of liquid drugs to people in need of therapeutic treatment. Many injection devices are capable of repeated setting and injection of either a fixed or a variable volume of drug upon operation of respective dose setting and dose expelling mechanisms in the device. Some injection devices are adapted to be loaded with a prefilled drug reservoir containing a volume of drug which is sufficient to provide for a number of injectable doses. When the reservoir is empty, the user replaces it with a new one and the injection device can thus be used again and again. Other injection devices are prefilled when delivered to the user and can only be used until the drug reservoir has been emptied, after which the device is discarded. The various injection devices comprise a dose expelling mechanism which typically expels the drug by advancing a piston in the reservoir using a motion controlled piston rod.

Some injection devices require the user to depress a push button a certain distance towards a housing to thereby manually cause the piston rod to pressurise the reservoir and advance the piston therein for expelling of a dose. The force which must be applied to the push button to perform such an operation is often not insignificant and may cause handling problems for people with reduced finger strength and/or dexterity.

Automatic injection devices offering automatic expelling of a dose of drug in response to a release of a cocked spring are popular because the spring, once released, provides all the energy needed to complete the injection. Such devices typically only require the user to apply a small, short-duration force to trigger the injection. The spring can either be arranged to be strained before each injection, or it can be pre-strained, e.g. by the manufacturer, to store energy sufficient to occasion an emptying of the drug reservoir in one or more injections.

WO 2006/045528 (Novo Nordisk A/S) discloses an automatic injection device utilising a helical torsion spring to provide energy for advancement of a piston in a drug cartridge. One end of the spring is connected to a housing and the other end is connected to a rotatable drive member which again is connected to a piston rod. The spring is strained during dose setting and released by removal of a locking member from the drive member.

Another example of an automatic injection device is found in WO 2008/053243 (Owen Mumford Ltd) which discloses use of a compression spring to provide a drive force for ejecting medicine from a loaded cartridge. The spring is arranged between a dose knob at the proximal end of the device and a lock element in the device casing.

A common drawback of the above mentioned injection devices is that the spring must be relatively long for the device to function satisfactorily. In the case of the torsion spring, which is adapted to release energy for rotation of the piston rod, it is imperative that the spring has sufficient capacity to rotate the drive member the number of revolutions required to deliver a volume of drug corresponding to the maximum settable dose. Further, the decrease of the torque transmitted by the spring during the injection as a consequence of the spring relaxing should be minimised to provide a relatively constant delivery profile and to ensure that sufficient power is left to expel the last portion of the dose. Thus, the spring must have a large number of coils and be partially strained in the end-of-dose state of the injection device. However, as indicated, this affects the configuration of the injection device in that it entails a significant axial dimension.

Correspondingly, in the case of the compression spring the spring must have sufficient capacity to push the lock and a piston rod forward to expel a volume of drug corresponding to the maximum settable dose, and the decrease of the force exerted by the spring during the injection as a consequence of the spring relaxing should be minimised to provide a relatively constant delivery profile and to ensure that sufficient power is left to expel the last portion of the dose.

Clearly, if one of the above mentioned springs is to be applied pre-strained in an injection device, with no possibility of re-straining, it must be even longer in order to have sufficient capacity to empty a full cartridge.

### SUMMARY OF THE INVENTION

It is an object of the invention to eliminate or reduce at least one of the drawbacks of the prior art, or to provide a useful alternative thereto. In particular, it is an object of the invention to provide a compact automatic drug delivery device, which is easy to produce, requires few components and has low manufacturing costs.

It is a further object of the invention to provide a drug delivery device which offers automatic drug delivery upon virtually effortless release of a spring as well as a relatively constant delivery profile over the whole range of deliverable doses.

In the disclosure of the present invention, aspects and embodiments will be described which will address one or more of the above objects and/or which will address objects apparent from the following text.

In a drug delivery device employing the principles of the present invention a drug expelling mechanism is powered by a dual layer spring. Compared to a conventional single layer spring the dual layer spring can have a similar capacity with a much reduced axial dimension, offering the possibility of a compact drug delivery device construction.

Thus, in one aspect of the invention a drug delivery device is provided comprising a drug expelling mechanism and a spring releasable during use of the drug delivery device to convey stored energy to the drug expelling mechanism. The spring comprises a first helical portion, or a first coil portion, capable of storing a first amount of energy, and a second helical portion, or a second coil portion, capable of storing a second amount of energy. The first helical portion and the second helical portion form one continuous piece, and the second helical portion surrounds at least a portion of the first helical portion.

The drug delivery device is adapted to expel drug from a drug reservoir which may be an integrated part of the drug delivery device, as is conventionally the case in so-called prefilled drug delivery devices, or which alternatively may be a separate attachable part, such as e.g. an exchangeable part, as is conventionally the case in so-called re-usable drug delivery devices.

The spring may be composed of a single uninterrupted piece of material, e.g. an uninterrupted piece of metal wire. Alternatively, the spring may be composed of a plurality of coalesced pieces of material, e.g. welded or otherwise bonded together to form a continuous spring structure.

In addition to the fact that a dual layer spring enables a greater flexibility in the design and dimensioning of the drug delivery device because the manufacturer can choose to prioritise a shorter device having a similar capacity as devices employing a conventional single layer spring, or a device of conventional length having a larger capacity, or something in between, a drug delivery device with a dual layer spring offers a more constant force profile, or torque profile depending on the particular type of spring, producing a more constant pressurisation of the drug reservoir over time and thus a more constant flow of drug to the body. Further, if made of a single uninterrupted piece of material, e.g. on modified coil machinery, the dual layer spring can be manufactured in a simple manner at only a small additional cost to that of a single layer spring.

The spring may further comprise a first spring end portion, being an end portion of the first helical portion, a second spring end portion, being an end portion of the second helical portion, and a transitional portion joining the first helical portion and the second helical portion.

The spring may be formed by winding a piece of wire of a certain length in an arbitrary (e.g. clockwise) direction about an axis to produce the first helical portion and then change the deformation angle and continue winding the wire in the same rotational direction (e.g. clockwise) back over the first helical portion to produce the second helical portion. Thereby, the first helical portion becomes an inner helix and the second helical portion becomes an outer helix.

The second helical portion may be wound directly upon the first helical portion or upon a shaped former, such as e.g. a cylinder, whereby it may be radially spaced apart from the first helical portion. Further, the first helical portion and the second helical portion may be concentric to minimise the radial dimension of a spring having a given capacity.

The respective axial extent of the first helical portion and the second helical portion may be chosen by the manufacturer in accordance with the desired properties of the spring vis-à-vis the drug delivery device. In order to decidedly benefit from the configuration of the spring with respect to a reduction of the axial dimension of the drug delivery device the axial extent of the second helical portion should preferably be between 50% and 150%, particularly between 50% and 100%, of the axial extent of the first helical portion.

The drug delivery device may further comprise a housing coupled with, or adapted to be coupled with, a variable volume drug reservoir, and the drug expelling mechanism may comprise an actuator displaceable relative to the housing to reduce the volume of the reservoir. During release of energy from the spring the actuator may be advanced through the housing, either directly by the spring or via a drive structure configured to convert a motor output by the spring to a displacement of the actuator. In either case, one of the first spring end portion and the second spring end portion may be arranged stationarily with respect to the housing during such release, while the other of the first spring end portion and the second spring end portion may be operatively coupled with the actuator.

The drug delivery device may be of the injection device type, e.g. having a configuration corresponding to that of a fountain pen. In that case the housing may comprise a circular cylindrical (or substantially circular cylindrical) wall within which the spring may be co-axially arranged.

The spring may be arranged in a pre-strained state in the drug delivery device that allows expelling of the entire contents of the reservoir without straining or re-straining of the spring by the user. Alternatively, the spring may be adapted to be strained by the user before each drug administration. Regardless, in conventional fashion, the spring may be arranged so as to be at least slightly strained in the end-of-dose state of the drug delivery device to ensure that it is capable of transmitting a sufficient force or torque to the actuator at the end of the drug expelling stroke to enable proper delivery also of the last portion of the dose.

The drug delivery device may further comprise a dose setting mechanism allowing the user to set a dose to be expelled from the reservoir. The spring may be operatively coupled with the dose setting mechanism and configured to undergo straining in response to an operation of the dose setting mechanism that increases the dose to be expelled and to undergo relaxation in response to an operation of the dose setting mechanism that decreases the dose to be expelled. Thereby, additional energy is being stored in the spring in correspondence with the size of the dose being set.

In particular embodiments of the invention one of the first helical portion and the second helical portion acts as a tension spring and the other of the first helical portion and the second helical portion acts as a compression spring, thereby enabling a cumulative force transmission to the drug expelling mechanism. For example, the first helical portion may be open wound, i.e. configured as a compression spring, and the second helical portion may be close wound, configured as a tension spring.

In other particular embodiments of the invention the first helical portion and the second helical portion are both close wound and act as torsion springs.

In a certain embodiment of the invention the drug delivery device comprises a) a housing extending along a longitudinal axis, b) a variable volume drug reservoir comprising an outlet portion (e.g. a drug cartridge comprising a slidable piston and an outlet covered by a penetrable septum), c) a torsion spring comprising a first spring end and a second spring end, the torsion spring being strained to bias the second spring end in a spring unwinding direction relative to the first spring end, d) a first spring holding member adapted to hold the first spring end, the first spring holding member being non-rotatable relative to the housing at least during a volume reduction of the reservoir, e) an activation button, f) a second spring holding member adapted to hold the second spring end, the second spring holding member being adapted to be transferred from a first state in which a rotation of the second spring holding member in the spring unwinding direction relative to the first spring holding member is prevented to a second state in which a rotation of the second spring holding member in the spring unwinding direction relative to the first spring holding member is allowed in response to an operation of the activation button, g) an actuator (e.g. a piston rod) for pressurising the drug reservoir, the actuator being rotationally coupled with the second spring holding member when the second spring holding member is in the second state, and h) an actuator guide structure (e.g. a nut member) configured to cause the actuator to pressurise the drug reservoir in response to the second spring holding member rotating in the spring unwinding direction, wherein the torsion spring further comprises a first torsion spring portion comprising the first spring end and a second torsion spring portion comprising the second spring end, the second torsion spring portion being wound about at least a portion of the first torsion spring portion, and the first torsion spring portion and the second torsion spring portion forming one continuous piece.

In another embodiment of the invention the drug delivery device comprises a) a housing extending along a longitudinal axis and having a window, b) a variable volume drug reservoir comprising an outlet portion (e.g. a drug cartridge comprising a slidable piston and an outlet covered by a penetrable septum), c) a torsion spring comprising a first spring end and a second spring end, d) a first spring holding member adapted to hold the first spring end, the first spring holding member being non-rotatable relative to the housing, e) a dose setting member for setting a dose to be expelled from the drug reservoir, f) a dose indicator configured for displacement relative to the window in response to an operation of the dose setting member, and g) a dose expelling mechanism comprising g1) an activation button, g2) a second spring holding member adapted to hold the second spring end, the second spring holding member being operatively coupled with the activation button and configured to translate relative to the housing from a first axial position in which the second spring holding member is prevented from rotating in a spring unwinding direction relative to the housing to a second axial position in which the second spring holding member is allowed to rotate in the spring unwinding direction relative to the housing in response to an operation of the activation button, g3) an actuator (e.g. a piston rod) for pressurising the drug reservoir, the actuator being rotationally coupled with the second spring holding member when the second spring holding member is in the second axial position, and g4) an actuator guide structure (e.g. a nut member) configured to cause the actuator to pressurise the drug reservoir in response to the second spring holding member rotating in the spring unwinding direction, wherein the torsion spring further comprises a first torsion spring portion comprising the first spring end and a second torsion spring portion comprising the second spring end, the second torsion spring portion being wound about at least a portion of the first torsion spring portion, and the first torsion spring portion and the second torsion spring portion forming one continuous piece.

It is noted that, within the scope of the invention, the spring is not limited to comprise two layers, but may further comprise a third helical portion being wound about at least a portion of the second helical portion, a fourth helical portion being wound about at least a portion of the third helical portion and so forth, each additional coil layer enabling further axial compactness.

In another aspect the invention provides a use of a spring in a drug delivery device for energising a drug expelling mechanism, the spring comprising an inner helical spring member and an outer helical spring member forming one continuous piece. The spring may be configured as described in the above and/or as disclosed below in connection with the description of exemplary embodiments. Particularly, the spring used in the drug delivery device may be composed of an uninterrupted piece of material.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 is a perspective view of a pre-strained combined compression/tension spring for use in a drug delivery device according to a first embodiment of the invention,
Fig. 2a is a longitudinal section view of a drug delivery device employing the combined compression/tension spring of Fig. 1,
Fig. 2b is a longitudinal section view of the drug delivery device after release of the spring,
Fig. 3 is a perspective view of a torsion spring for use in a drug delivery device according to a second embodiment of the invention,
Fig. 4 is a longitudinal and partially sectional view of a drug delivery device employing the torsion spring of Fig. 3, and
Fig. 5 is a perspective, partially sectional view showing the mounting of the torsion spring.

In the figures like structures are mainly identified by like reference numerals.

### DESCRIPTION OF EXEMPLARY EMBODIMENTS

When in the following relative expressions, such as "upward" and "downward", are used, these refer to the appended figures and not necessarily to an actual situation of use. The shown figures are schematic representations for which reason the configuration of the different structures as well as their relative dimensions are intended to serve illustrative purposes only.

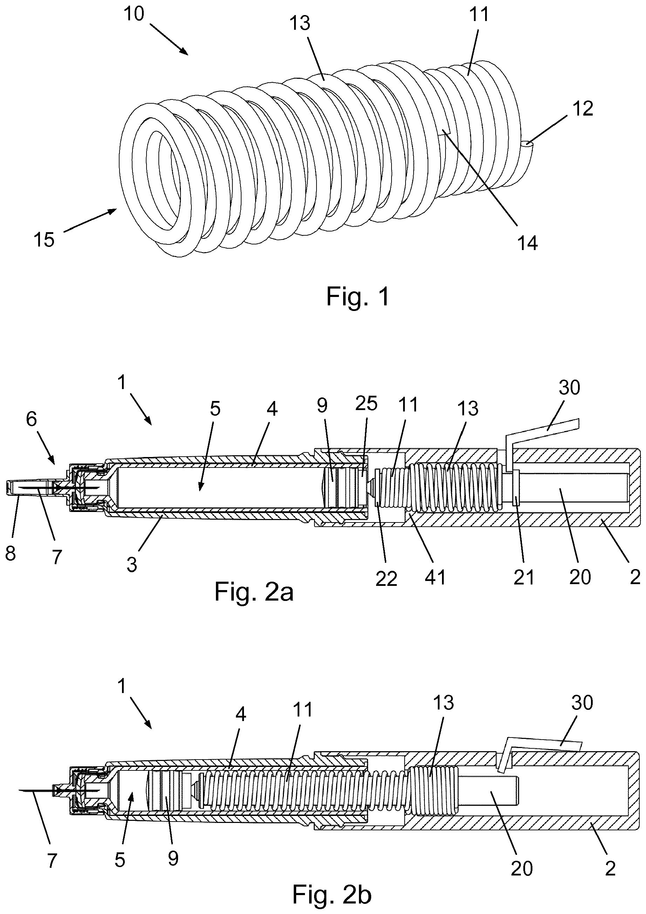

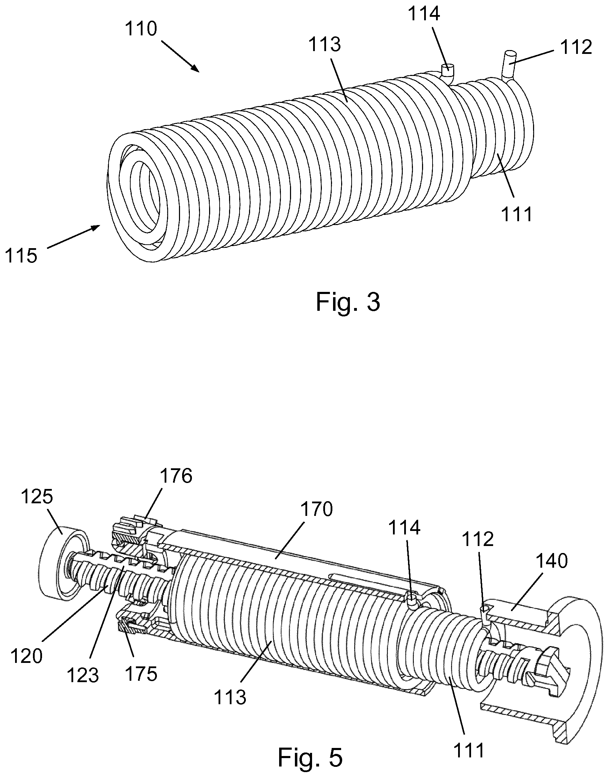

Figs. 1 and 2 relate to a first exemplary embodiment of the invention in which a first type of dual layer spring is incorporated pre-strained in a single shot injection device capable of delivering only one dose of drug, whereas figs. 3-5 relate to a second exemplary embodiment of the invention in which a second type of dual layer spring is incorporated in a multi-dose injection device capable of setting and delivering a plurality of doses of drug. It is understood that each of the two types of dual layer springs are equally applicable in the other injection device type as well as in further types of drug delivery devices.

Fig. 1 shows a dual layer combined compression/tension spring 10 in a pre-strained condition. The spring 10 has an inner coil 11 which is wound from an inner spring end 12 to a transitional portion 15 and an outer coil 13 which is wound back over the inner coil 11 from the transitional portion 15 to an outer spring end 14. The inner coil 11 is an open wound (although it appears close wound because the spring 10 is depicted in its tensioned state) compression spring and the outer coil 13 is a close wound tension spring.

Fig. 2a is a longitudinal section view of an injection device 1 comprising the spring 10, certain components, most notably the spring 10, being shown non-sectioned. The injection device 1 comprises a housing 2, a cartridge holder 3 holding a cartridge 4, and a drug expelling mechanism powered by the spring 10. A needle hub 6 carrying an injection needle 7 is attached to the distal end of the cartridge holder 3 in a manner that allows the injection needle 7 to extend through a penetrable septum sealing the distal end of the cartridge 4. A sealing piston 9 is slidably mounted at the opposite end of the cartridge 4, providing for a variable volume chamber 5 capable of holding a liquid drug. The piston 9 is adapted to be advanced in the cartridge 4 by a piston rod 20 forming part of the drug expelling mechanism. The piston 9 is abutted by a piston rod foot 25 which is rigidly coupled to the piston rod 20.

Fig. 2a shows the injection device 1 in a pre-use state, a removable needle cap 8 covering an exterior portion of the injection needle 7. The pre-strained spring 10 is mounted such that the outer spring end 14 contacts a spring base 41 and the inner spring end 12 contacts a distal flange 22 on the piston rod 20. Further, a latch 30 engages a proximal flange 21 on the piston rod 20, thereby providing a cocked drug expelling mechanism.

Fig. 2b shows the injection device 1 after release of the drug delivery mechanism. The user has pressed the arm of the latch 30 against the housing 2 to set the piston rod 20 free, and the spring 10 has consequently conveyed energy stored therein to the piston rod 20 to urge the piston 9 in the distal direction for collapse of the chamber 5. The two layers of the spring 10 have each contributed an amount of the total energy conveyed to the drug expelling mechanism. During the relaxation of the spring 10 the inner coil 11 expands while the outer coil 13 simultaneously contracts, translating the transitional portion 15 towards the spring base 41. A resulting force consisting of superpositioned force contributions from the inner coil 11 and the outer coil 13 is thus transferred to the distal flange 22 and converted to a resulting stroke of the piston rod 20.

Fig. 3 shows a dual layer torsion spring 110 adapted for use in a drug delivery device according to another embodiment of the invention. The spring 110 has an inner coil 111 which is wound from an inner spring end 112 to a transitional portion 115 and an outer coil 113 which is wound back over the inner coil 111 from the transitional portion 115 to an outer spring end 114. Both the inner coil 111 and the outer coil 113 are close wound, and both the inner spring end 112 and the outer spring end 114 are formed to enable interaction with respective other components for transfer of a torque therebetween. The spring 110 is thus adapted to store and deliver rotational energy, and its capacity in terms of torque and stroke is almost twice that of a single layer torsion spring of the same length and diameter as the inner coil 111.

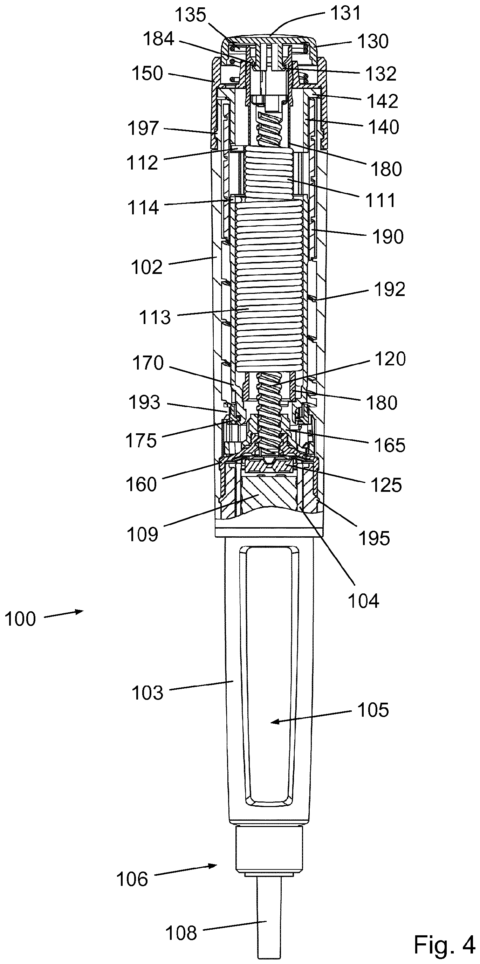

Fig. 4 is a partially sectional view of an injection device 100 employing the spring 110. The injection device 100 comprises a housing 102, a cartridge holder 103 holding a cartridge 104, and a drug expelling mechanism powered by the spring 110. The cartridge holder 103 is firmly attached to the housing 102 via a circumferential ridge 195. A needle hub 106 carrying an injection needle, which in the figure is covered by a needle cap 108, is removably attached to the distal end of the cartridge holder 103 in a manner that allows the injection needle to extend through a penetrable septum sealing the distal end of the cartridge 104. A sealing piston 109 is slidably mounted at the opposite end of the cartridge 104, providing for a variable volume chamber 105 capable of holding a liquid drug. The piston 109 is adapted to be advanced in the cartridge 104 by a piston rod 120 forming part of the drug expelling mechanism. The piston 109 is abutted by a piston rod foot 125 which is rigidly coupled to the piston rod 120. The piston rod 120 is threadedly engaged with a nut 160, which is fixedly mounted in the housing 102, and rotationally locked to a piston rod guide 165.

A dose dial 150 is rotatably mounted on the proximal end of the housing 102 over a circumferential ridge 197, which prevents relative axial movement between the two. An inner geometry of the dose dial 150 ensures rotational locking engagement with a tube 180 which extends through the spring 110. The tube 180 is at its proximal end portion provided with a radial constriction 184 formed for engagement with a flange 132 of an injection button 130 to prevent upward movement of the injection button 130 relative to the tube 180. The injection button 130 is user operable, by application of a force to a push face 131, to activate the drug expelling mechanism, as will be explained in further detail below. The underside of the push face 131 abuts the proximal most portion of the tube 180, thereby preventing downward movement of the injection button 130 relative to the tube 180. The injection button 130 and the tube 180 are thus forced to undergo joint axial movements. A compression spring 135 is arranged between the underside of the push face 131 and the dose dial 150 to bias the injection button 130, and thereby the tube 180, proximally relative to the housing 102.

A helical track 192 is formed on the inner wall of the housing 102 for engagement with a scale drum 190. The scale drum 190 carries a plurality of dose related ciphers (not visible) and is adapted to be positioned along the helical track 192 during dose setting and to travel a portion of the helical track 192 during dose expelling, as will also be described in further detail below. In its proximal most position the scale drum 190 abuts a flange 142 of a spring base 140. The spring base 140 is axially and rotationally locked with respect to the housing 102 and serves to retain the inner spring end 112. A number of axial tracks (not visible) are provided along the inner wall of the scale drum 190 for splined engagement with a ratchet 170 which retains the outer spring end 114. The ratchet 170 is axially and rotationally locked to the tube 180 and axially locked to a clutch 175. The clutch 175 is in turn rotationally locked to the housing 102 during dose setting, via engagement with a plurality of axial splines 193 circumferentially distributed on the inner wall thereof, and decoupled from the housing 102 during dose expelling.

Fig. 5 shows isometrically the mounting of the spring 110 and details the respective connections between the inner spring end 112 and the spring base 140 and between the outer spring end 114 and the ratchet 170. The figure further shows an axial track 123 through the thread of the piston rod 120. The axial track 123 is adapted for engagement by a key (not shown) in the piston rod guide 165, ensuring the rotational interlocking of the piston rod 120 and the piston rod guide 165. Also seen are a plurality of teeth 176 circumferentially distributed on the outer surface of the clutch 175. The teeth 176 are adapted for interaction with the splines 193 on the inner wall of the housing 102.

In the following an operation of the injection device 100 will be described. Fig. 4 shows the injection device 100 in a pre-use state ready for setting of a dose to be delivered from the chamber 105. In this state the scale drum 190 is in zero dose position, abutting the flange 142, and a zero dose indication is displayed in a window (not shown) in the housing 102. To set the dose the user grips the injection device 100 and turns the dose dial 150 until the desired dose is displayed in the window. The rotation of the dose dial 150 is transferred to the tube 180 and further on to the ratchet 170. Thereby, the outer spring end 114 will be angularly displaced relative to the inner spring end 112, which is retained by the fixed spring base 140, and the spring 110 will consequently become further strained.

Due to the splined relationship between the ratchet 170 and the scale drum 190 a clockwise rotation of the ratchet 170 (seen from a proximal viewpoint) causes the scale drum 190 to travel distally along the helical track 192, while a counter-clockwise rotation of the ratchet 170 causes the scale drum 190 to travel the helical track 192 in the opposite direction. This means that the user will see the cipher in the window increase as the dose dial 150 is turned clockwise and decrease as the dose dial 150 is turned counter-clockwise.

In the shown dose setting state of the injection device 100 the clutch 175 is rotationally locked to the housing 102 due to an interaction between the teeth 176 and the splines 193. The relative rotation between the ratchet 170 and the clutch 175 during the dose setting is governed by an internal ratchet and pawl mechanism (not visible) that prevents the ratchet 170 from rotating counter-clockwise when subjected to a torque from the strained spring 110. Thereby, the angular position of the ratchet 170 relative to the housing 102 as obtained during the dose setting is maintained against the bias of the strained spring 110 when the user lets go of the dose dial 150. However, the internal ratchet and pawl mechanism is configured to allow counter-clockwise rotation of the ratchet 170 relative to the clutch 175 when the tube 180 is subjected to a torque from the dose dial 150 in connection with the dose dial 150 being turned counter-clockwise to reduce the set dose.

To eject the set dose through the injection needle the user depresses the injection button 130 against the biasing force from the compression spring 135. The downward movement of the injection button 130 is transferred to the tube 180 and in turn to the ratchet 170 and the clutch 175. As the clutch 175 moves downwards in the housing 102 the teeth 176 firstly engage with the piston rod guide 165 and subsequently disengage from the splines 193, allowing the spring 110 to unwind and rotate the ratchet 170 counter-clockwise with respect to the housing 102. The ratchet 170 slaves the clutch 175 and the piston rod guide 165 in the rotational motion, and the splined relationship between the piston rod guide 165 and the piston rod 120 causes the latter to rotate jointly and become advanced helically through the nut 160, whereby the piston rod foot 125 displaces the piston 109 distally and drug is expelled through the injection needle (the needle cap 108 having been removed).

The counter-clockwise rotation of the ratchet 170 also causes the scale drum 190 to travel the helical track 192 proximally from the dose indicating position it was left in during dose setting to the initial zero dose position where it meets the flange 142 and is brought to a stop. When the scale drum 190 can no longer rotate counter-clockwise the ratchet 170 stops rotating and the distal movement of the piston rod 120 is consequently also halted, thereby ending the dose ejection.

When the user subsequently removes the force from the push face 131 the compression spring 135 urges the injection button 130 proximally, whereby the tube 180 is lifted by the flange 132 interacting with the radial constriction 184. Because the ratchet 170 is axially locked to the tube 180 and the clutch 175, this proximal displacement of the tube 180 is transferred to the clutch 175 which is moved out of engagement with the piston rod guide 165 and back into engagement with the splines 193 in the housing 102. The clutch 175 is thereby again prevented from rotation relative to the housing 102, and the injection device 100 is thus automatically returned to the dose setting state.

The particular configuration of the spring 110 allows for an axially compact construction of the injection device 100 while ensuring a uniform pressurisation of the chamber 105 by the piston rod 120 and thus a steady flow of drug through the injection needle during the entire dose ejection.

## Claims

1. A drug delivery device (1, 100) comprising a drug expelling mechanism and a spring (10, 110) releasable during use of the drug delivery device (1, 100) to convey stored energy to the drug expelling mechanism, wherein the spring (10, 110) comprises a first helical portion (11, 111) and a second helical portion (13, 113) wound about at least a portion of the first helical portion (11, 111), and wherein the first helical portion (11, 111) and the second helical portion (13, 113) form one continuous piece.

2. A drug delivery device according to claim 1, wherein the spring (10, 110) is formed of a single uninterrupted piece of material.

3. A drug delivery device according to claim 1 or 2, wherein the first helical portion (11, 111) and the second helical portion (13, 113) are concentric.

4. A drug delivery device according to any of claims 1 - 3, wherein the first helical portion (11, 111) is wound about an axis and has a first axial extent, and the second helical portion (13, 113) is wound about the first helical portion (11, 111) and has a second axial extent which is at least 50% of the first axial extent.

5. A drug delivery device according to any of claims 1 - 4, wherein the rotational direction of the first helical portion (11, 111) and the second helical portion (13, 113) is the same.

6. A drug delivery device according to any of claims 1 - 5, further comprising a housing (2, 102), wherein the drug expelling mechanism comprises an actuator (20, 120) adapted to interact with a movable wall of a drug reservoir, wherein the spring (10, 110) further comprises a first spring end portion (12, 112) which is an end portion of the first helical portion (11, 111) and a second spring end portion (14, 114) which is an end portion of the second helical portion (13, 113), and wherein one of the first spring end portion (12, 112) and the second spring end portion (14, 114) is arranged stationarily with respect to the housing (2, 102) during release of energy from the spring (10, 110), and the other of the first spring end portion (12, 112) and the second spring end portion (14, 114) is operatively coupled with the actuator (20, 120) during release of energy from the spring (10, 110).

7. A drug delivery device according to claim 6, wherein the housing (2, 102) is circular cylindrical and the spring element (10, 110) is arranged concentrically with the housing (2, 102).

8. A drug delivery device according to claims 6 or 7, wherein the spring (10, 110) is pre-strained and has sufficient potential energy stored to empty the drug reservoir.

9. A drug delivery device according to any of claims 1 - 7, further comprising a dose setting mechanism for selective setting of a dose to be expelled by the drug expelling mechanism, wherein the spring (10, 110) is operatively coupled with the dose setting mechanism and configured to store energy in response to a dose increasing dose setting operation.

10. A drug delivery device according to any of claims 1 - 9, wherein one of the first helical portion (11, 111) and the second helical portion (13, 113) acts as a tension spring and the other of the first helical portion (11, 111) and the second helical portion (13, 113) acts as a compression spring.

11. A drug delivery device according to any of claims 1 - 8, wherein the first helical portion (11, 111) and the second helical portion (13, 113) both act as a torsion spring.

12. Use of a spring (10, 110) in a drug delivery device (1, 100) for energising a drug expelling mechanism, the spring (10, 110) comprising an inner helical spring member and an outer helical spring member forming one continuous piece.

## Patentansprüche

1. Medikamenten-Verabreichungsvorrichtung (1, 100), umfassend einen Medikamentenausstoßmechanismus und eine Feder (10, 110), die während der Verwendung der Medikamenten-Verabreichungsvorrichtung (1, 100) freisetzbar ist, um gespeicherte Energie an den Medikamentenausstoßmechanismus zu übertragen, wobei die Feder (10, 110) einen ersten spiralförmigen Abschnitt (11, 111) und einen zweiten spiralförmigen Abschnitt (13, 113), der um mindestens einen Teil des ersten spiralförmigen Abschnitts (11, 111) gewickelt ist, umfasst und wobei der erste spiralförmige Abschnitt (11, 111) und der zweite spiralförmige Abschnitt (13, 113) ein durchgehendes Teil bilden.

2. Medikamenten-Verabreichungsvorrichtung nach Anspruch 1, wobei die Feder (10, 110) aus einem einzigen ununterbrochenen Materialteil gebildet ist.

3. Medikamenten-Verabreichungsvorrichtung nach Anspruch 1 oder 2, wobei der erste spiralförmige Abschnitt (11, 111) und der zweite spiralförmige Abschnitt (13, 113) konzentrisch sind.

4. Medikamenten-Verabreichungsvorrichtung nach einem der Ansprüche 1 - 3, wobei der erste spiralförmige Abschnitt (11, 111) um eine Achse gewickelt ist und eine erste axiale Erstreckung hat, und der zweite spiralförmige Abschnitt (13, 113) um den ersten spiralförmigen Abschnitt (11, 111) gewickelt ist und eine zweite axiale Erstreckung hat, die mindestens 50% der ersten axialen Erstreckung beträgt.

5. Medikamenten-Verabreichungsvorrichtung nach einem der Ansprüche 1 - 4, wobei die Rotationsrichtung des ersten spiralförmigen Abschnitts (11, 111) und des zweiten spiralförmigen Abschnitts (13, 113) dieselbe ist.

6. Medikamenten-Verabreichungsvorrichtung nach einem der Ansprüche 1 - 5, ferner umfassend ein Gehäuse (2, 102), wobei der Medikamentenausstoßmechanismus einen Aktuator (20, 120) umfasst, der geeignet ist, mit einer beweglichen Wand eines Medikamentenreservoirs zusammenzuwirken, wobei die Feder (10, 110) ferner einen ersten Federendabschnitt (12, 112), der ein Endabschnitt des ersten spiralförmigen Abschnitts (11, 111) ist, und einen zweiten Federendabschnitt (14, 114), der ein Endabschnitt des zweiten spiralförmigen Abschnitts (13, 113) ist, umfasst und wobei der erste Federendabschnitt (12, 112) oder der zweite Federendabschnitt (14, 114) während des Freisetzens von Energie aus der Feder (10, 110) feststehend bezüglich des Gehäuses (2, 102) angeordnet ist und der jeweils andere des ersten Federendabschnitts (12, 112) und des zweiten Federendabschnitts (14, 114) während des Freisetzens von Energie aus der Feder (10, 110) mit dem Aktuator (20, 120) wirkgekoppelt ist.

7. Medikamenten-Verabreichungsvorrichtung nach Anspruch 6, wobei das Gehäuse (2, 102) kreiszylindrisch ist und das Federelement (10, 110) konzentrisch mit dem Gehäuse (2, 102) angeordnet ist.

8. Medikamenten-Verabreichungsvorrichtung nach Anspruch 6 oder 7, wobei die Feder (10, 110) vorgespannt ist und über hinreichende potenzielle gespeicherte Energie verfügt, um das Medikamentenreservoir zu leeren.

9. Medikamenten-Verabreichungsvorrichtung nach einem der Ansprüche 1 - 7, ferner umfassend einen Dosiseinstellmechanismus zum gezielten Einstellen einer durch den Medikamentenausstoßmechanismus auszustoßenden Dosis, wobei die Feder (10, 110) mit dem Dosiseinstellmechanismus wirkgekoppelt und dazu konfiguriert ist, als Reaktion auf einen dosiserhöhenden Dosiseinstellvorgang Energie zu speichern.

10. Medikamenten-Verabreichungsvorrichtung nach einem der Ansprüche 1 - 9, wobei der erste spiralförmige Abschnitt (11, 111) oder der zweite spiralförmige Abschnitt (13, 113) als eine Zugfeder wirkt und der jeweils andere des ersten spiralförmigen Abschnitts (11, 111) und des zweiten spiralförmigen Abschnitts (13, 113) als eine Druckfeder wirkt.

11. Medikamenten-Verabreichungsvorrichtung nach einem der Ansprüche 1 - 8, wobei sowohl der erste spiralförmige Abschnitt (11, 111) als auch der zweite spiralförmige Abschnitt (13, 113) als eine Zugfeder wirken.

12. Verwendung einer Feder (10, 110) in einer Medikamenten-Verabreichungsvorrichtung (1, 100) zum Antreiben eines Medikamentenausstoßmechanismus, wobei die Feder (10, 110) ein inneres spiralförmiges Federelement und ein äußeres spiralförmiges Federelement umfasst, die ein durchgehendes Teil bilden.

## Revendications

1. Dispositif (1, 100) d'administration de médicament comportant un mécanisme d'expulsion de médicament et un ressort (10, 110) libérable durant l'utilisation du dispositif (1, 100) d'administration de médicament pour transférer de l'énergie emmagasinée au mécanisme d'expulsion de médicament, dans lequel le ressort (10, 110) comporte une première partie (11, 111) hélicoïdale et une seconde partie (13, 113) hélicoïdale enroulée autour d'au moins une partie de la première partie (11, 111) hélicoïdale, et dans lequel la première partie (11, 111) hélicoïdale et la seconde partie (13, 113) hélicoïdale forment une pièce continue.

2. Dispositif d'administration de médicament selon la revendication 1, dans lequel le ressort (10, 110) est constitué d'une seule pièce ininterrompue de matériau.

3. Dispositif d'administration de médicament selon la revendication 1 ou 2, dans lequel la première partie (11, 111) hélicoïdale et la seconde partie (13, 113) hélicoïdale sont concentriques.

4. Dispositif d'administration de médicament selon l'une quelconque des revendications 1 à 3, dans lequel la première partie (11, 111) hélicoïdale est enroulée autour d'un axe et présente une première étendue axiale, et la seconde partie (13, 113) hélicoïdale est enroulée autour de la première partie (11, 111) hélicoïdale et présente une seconde étendue axiale qui représente au moins 50 % de la première étendue axiale.

5. Dispositif d'administration de médicament selon l'une quelconque des revendications 1 à 4, dans lequel la direction de rotation de la première partie (11, 111) hélicoïdale et de la seconde partie (13, 113) hélicoïdale est la même.

6. Dispositif d'administration de médicament selon l'une quelconque des revendications 1 à 5, comportant en outre un logement (2, 102), dans lequel le mécanisme d'expulsion de médicament comporte un actionneur (20, 120) apte à exercer une interaction avec une paroi mobile d'un réservoir de médicament, dans lequel le ressort (10, 110) comporte en outre une première partie (12, 112) d'extrémité de ressort qui est une partie d'extrémité de la première partie (11, 111) hélicoïdale et une seconde partie (14, 114) d'extrémité de ressort qui est une partie d'extrémité de la seconde partie (13, 113) hélicoïdale, et dans lequel l'une de la première partie (12, 112) d'extrémité de ressort et de la seconde partie (14, 114) d'extrémité de ressort est agencée de manière fixe par rapport au logement (2, 102) durant la libération d'énergie depuis le ressort (10, 110), et l'autre de la première partie (12, 112) d'extrémité de ressort et de la seconde partie (14, 114) d'extrémité de ressort est accouplée de manière fonctionnelle à l'actionneur (20, 120) durant la libération d'énergie depuis le ressort (10, 110).

7. Dispositif d'administration de médicament selon la revendication 6, dans lequel le logement (2, 102) est cylindrique circulaire et l'élément (10, 110) ressort est agencé concentriquement par rapport au logement (2, 102).

8. Dispositif d'administration de médicament selon les revendications 6 ou 7, dans lequel le ressort (10, 110) est précontraint et a une énergie potentielle emmagasinée suffisante pour vider le réservoir de médicament.

9. Dispositif d'administration de médicament selon l'une quelconque des revendications 1 à 7, comportant en outre un mécanisme de réglage de dose permettant de régler de manière sélective une dose devant être expulsée par le mécanisme d'expulsion de médicament, dans lequel le ressort (10, 110) est accouplé de manière fonctionnelle au mécanisme de réglage de dose et configuré pour emmagasiner de l'énergie en réponse à une opération de réglage de dose augmentant une dose.

10. Dispositif d'administration de médicament selon l'une quelconque des revendications 1 à 9, dans lequel l'une de la première partie (11, 111) hélicoïdale et de la seconde partie (13, 113) hélicoïdale fait office de ressort de tension et l'autre de la première partie (11, 111) hélicoïdale et de la seconde partie (13, 113) hélicoïdale fait office de ressort de compression.

11. Dispositif d'administration de médicament selon l'une quelconque des revendications 1 à 8, dans lequel la première partie (11, 111) hélicoïdale et la seconde partie (13, 113) hélicoïdale font les deux office de ressort de torsion.

12. Utilisation d'un ressort (10, 110) dans un dispositif (1, 100) d'administration de médicament permettant de mettre sous tension un mécanisme d'expulsion de médicament, le ressort (10, 110) comportant un élément ressort hélicoïdal interne et un élément ressort hélicoïdal externe formant une pièce continue.
